# NEUE EUROPÄISCHE PATENTSCHRIFT

(11) **EP 2 442 773 B2**
(45) Veröffentlichungstag und Bekanntmachung des Hinweises auf die Entscheidung über den Einspruch: **24.08.2016**
(45) Hinweis auf die Patenterteilung: 15.05.2013
(21) Anmeldenummer: 10744682.5
(22) Anmeldetag: 16.06.2010
(51) Int. Cl.: A61F 13/06, A61F 13/08

(54) **STÜTZBANDAGE**
COMPRESSION BANDAGE
BANDE DE CONTENTION

(30) Priorität: 16.06.2009 DE 102009025415; 22.10.2009 DE 102009050383
(43) Veröffentlichungstag der Anmeldung: 25.04.2012
(60) Teilanmeldung: 13001669.4
(73) Patentinhaber: Otto Bock HealthCare GmbH, 37115 Duderstadt (DE)
(72) Erfinder: VOLLBRECHT, Matthias, 37412 Herzberg (DE); REINHARDT, Holger, 47906 Kempen (DE); BRÜGGEMANN, Gert-Peter, 50858 Köln (DE); ELLERMANN, Andree, 76275 Ettlingen (DE); BEST, Raymond, 70180 Stuttgart (DE); ALBASINI, Alfio, 6595 Riazzino, Ticino (CH); GÖSELE-KOPPENBURG, Andreas, 79540 Lörrach (DE); LIEBAU, Christian, 38126 Braunschweig (DE)
(74) Vertreter: Stornebel, Kai
(86) Internationale Anmeldenummer: PCT/EP2010/003624
(87) Internationale Veröffentlichungsnummer: WO 2010/145818

(56) Entgegenhaltungen:
- EP-A1- 1 338 260
- EP-B1- 0 492 328
- EP-B1- 1 634 555
- EP-B1- 2 117 366
- WO-A1-2007/018484
- DE-A1- 3 902 434
- DE-A1- 19 824 649
- DE-A1- 19 940 019
- DE-C1- 3 441 496
- DE-C2- 4 419 260
- DE-T2- 69 802 594
- DE-U1- 20 005 661
- DE-U1- 20 005 742
- US-A- 4 653 492
- US-A- 5 387 183
- US-A1- 2005 033 216
- US-A1- 2008 269 655
- US-B1- 6 863 657
- Auszug aus "Kompendium der lymphologischen Kompresssionsversorgung", Blundesfachschule für Orthopädie-Technik, Seiten 70, 71 und 94,95
- Fotografische Darstellungen: Figur 1 (elastisches Hosenband, geführt im Saum einer Jeanshose), Figur 2 (ein Brutgurt an einem Wanderrucksack)

## Beschreibung

Die Erfindung betrifft eine Stützbandage mit einem Grundkörper aus einem elastischen Textilmaterial, der eine dem Körper eines Bandagennutzers zugewandte Innenseite und eine dem Körper des Bandagennutzer abgewandte Außenseite aufweist, mit einer auf der Innenseite angeordneten rutschhemmenden Beschichtung.

Eine solche Stützbandage dient zur Unterstützung eines Gelenkes oder einer Gliedmaße, beispielsweise einem Oberschenkel oder Unterschenkel. Stützbandagen können am Sprunggelenk, am Knie sowie an den Gelenken der oberen Extremitäten angelegt werden, ebenfalls ist es möglich, solche Stützbandagen entlang von Muskelsträngen anzuordnen, ohne Gelenke zu überbrücken. Die Stützbandagen werden im Rahmen von Rehabilitationsmaßnahmen getragen, um bei einer Verletzung den Heilungsprozess zu unterstützen und ggf. weiterhin eine sportliche Aktivität zu ermöglichen. Darüber hinaus können Stützbandagen zur Linderung von Schmerzen bei degenerativen Erkrankungen eingesetzt werden. Ein weiterer Einsatzzweck der Stützbandagen kann sein, dass eine Mehrdurchblutung in dem Muskelgewebe durch Ausübung eines gleichmäßigen Druckes erreicht wird. Die Mehrdurchblutung führt zur besseren Versorgung der Muskulatur mit sauerstoffreichem Blut, wodurch eine Leistungsverbesserung erreicht werden kann. Durch die Anwendung von Stützbandagen kann die Propriozeption verbessert werden. Durch Druck von außen werden die Rezeptoren angeregt, was zu einer verbesserten Koordination und einem höheren Aktivitätsniveau der Muskulatur führt.

Die DE 40 91 302 T1 beschreibt eine Stützbandage mit einem Hauptteil, der aus einem elastischen und einem nicht dehnbaren Material oder nur aus einem elastischen Material besteht. Ein Teil des elastischen Materials ist imprägniert, um die Elastizität bereichsweise zu verändern.

Die DE 3902434 A1 betrifft eine Stützbandage für Gliedmaßen und/oder Gelenke, die aus einem elastischen Textilmaterial besteht, bei der auf der mit der Haut in Berührung kommenden Innenseite des elastischen Textilmaterials Antirutschelemente aus Silikonmaterial angeordnet sind. Die Antirutschelemente können als einzelne Flecke ausgebildet sein; die Anordnung auf der Innenseite der Stützbandage ist nahezu beliebig.

Die WO 2007/018484 A1 beschreibt eine Stützbandage mit einem Grundkörper aus einem elastischen Textilmaterial mit einer rutschhemmenden Beschichtung auf der Innenseite. Ein Stützgurt in Gestalt einer Patellaunterstützung ist vorgesehen, der über einen Klettverschluss geschlossen werden kann. Auf der Innenseite des Gurtes ist eine Abstützung oder eine Anlage vorgesehen, die eine mediale und laterale Anlage des Gurtes unmittelbar im Patellabereich vermeidet.

Die US 6,863,657 B1 betrifft eine Patellaunterstützung mit einem textilen Hülsenkörper, an dessen Außenseite Spanngurte über einen Klettverschluss in variablen Positionen festgelegt werden können.

Die US 2005/0033216 A1 beschreibt einen Grundkörper aus elastischem Neopren. Der Grundkörper kann über einen Klettverschluss um einen Knöchel herumgelegt und fixiert werden. Ein Stützgurt ist an der Außenseite angenäht und kann auf der Außenseite des Grundkörpers über einen Klettverschluss fixiert werden.

Aufgabe der vorliegenden Erfindung ist es, eine verbesserte Stützbandage bereitzustellen, mit der der Stützeffekt individuell einstellbar und an die jeweiligen körperlichen Eigenschaften sowie an Veränderungen der Bandage oder der Anforderungen an die Bandage anpassbar ist.

Erfindungsgemäß wird diese Aufgabe durch eine Stützbandage mit den Merkmalen des Hauptanspruches gelöst. Vorteilhafte Ausgestaltungen und Weiterbildungen der Erfindung sind in den Unteransprüchen aufgeführt.

Die erfindungsgemäße Stützbandage mit einem Grundkörper aus einem elastischen Textilmaterial, der eine dem Körper eines Bandagennutzers zugewandte Innenseite und an dem Körper des Bandagennutzers angewandte Außenseite aufweist, mit einer auf der Innenseite angeordneten rutschhemmenden Beschichtung, sieht vor, dass die Stützbandage zumindest einen Stützgurt aufweist, der auf der Außenseite des Grundkörpers über Formschlusselemente reversibel angeordnet ist und dass auf der der Hautoberfläche zugewandten Innenseite der Stützgurt eine rutschhemmende Beschichtung aufweist. Durch die Anordnung eines Stützgurtes auf der Außenseite des Grundkörpers ist es möglich, individuell diejenigen Bereiche mit einer erhöhten elastischen Rückstellkraft zu beaufschlagen, die eine erhöhte Kompression benötigen. Dadurch ist es möglich, die Stützbandage außerordentlich flexibel einzusetzen und bei unterschiedlichen Indikationen durch eine unterschiedliche Anordnung des Stützgurtes oder mehrerer Stützgurte auf der Außenseite der Stützbandage eine individuelle Anpassung an den jeweiligen Patienten durchzuführen. Durch die reversible Anordnung des Stützgurtes auf der Außenseite über Formschlusselemente ist es möglich, den Stützgurt individuell einzustellen und ihn darüber hinaus nach dem Anlegen und Tragen der Stützbandage in seiner Anordnung relativ zu dem Grundkörper zu variieren, beispielsweise um bei nachlassender Rückstellkraft aufgrund der Erwärmung der Bandage oder Ermüdung der elastischen Elemente innerhalb des Grundkörpers die ursprünglich eingestellte Spannung aufrecht zu erhalten. Die Formschlusselemente selbst können reversibel auf der Außenseite des Grundkörpers angeordnet sein, so dass Bereiche, die zur Festlegung der Stützgurte besonders geeignet sind, individuell festgelegt werden können. Neben einer reversiblen Festlegung der Formschlusselemente oder Bereichen von Formschlusselementen können diese auch individuell festgelegt und fixiert werden, beispielsweise nach der Anpassung der Bandage an den Nutzer durch einen Therapeuten, der die Formschlusselemente positioniert und anschließend fixiert, beispielsweise durch Annähen, Ankleben oder Anschweißen.

Der Stützgurt kann aus nur einem unelastischen Abschnitt bestehen, so dass eine feste Anlage und eine Versteifung der Stützbandage realisiert werden kann. Der flexible, unelastische Stützgurt wird an dem Grundkörper fixiert und stabilisiert den Bereich, an dem der Grundkörper anliegt, beispielsweise ein Gelenk. Unelastisch ist dabei so zu verstehen, dass eine makroskopische elastische Verformung des Stützgurtmaterials nicht vorgesehen ist, die jedem Material innewohnende Elastizität ist als unvermeidlich hinzunehmen.

Eine Alternative sieht vor, dass der Stützgurt einen parallel zu dem unelastischen Abschnitt angeordneten elastischen Abschnitt aufweist und der unelastische Abschnitt an dem elastischen Abschnitt befestigt ist. Über den flexiblen, unelastischen Abschnitt ist es möglich, eine hohe Kompression auf den Grundkörper und damit auf den Nutzer der Stützbandage auszuüben. Der parallel dazu angeordnete elastische Abschnitt ermöglicht es, eine gewisse Flexibilität in den Stützgurt einzubauen, um der elastischen Dehnung des Grundkörpers folgen zu können. Der parallel angeordnete unelastische Abschnitt stellt eine Dehnungsbegrenzung dar, die gleichzeitig als Überlastsicherung eingesetzt werden kann. Die Elastizität der Stützgurte kann individuell ausgewählt werden, so dass auch unterschiedliche Elastizitäten der Stützgurte an einer Bandage vorhanden sein können. Die Auswahl der jeweils benötigten Elastizität erfolgt entsprechend den Anforderungen des Bandagennutzers oder eines Therapeuten, wobei die Elastizität von Null, also im Wesentlichen unelastisch, bis nahezu unendlich einstellbar ist.

Der unelastische Abschnitt kann permanent an dem elastischen Abschnitt fixiert sein und eine Länge aufweisen, die von der Länge des ungedehnten elastischen Abschnittes verschieden ist. Je nach Ausgestaltung der Längenverhältnisse ist es dadurch möglich, einen reinen unelastischen Gurt zu haben, der auf dem Grundkörper aufgebracht wird und beispielsweise eine Fixierung der Kompression durch den Grundkörper ermöglicht. Soll dann die elastische Wirkung des Stützgurtes ausgenutzt werden, kann der unelastische Abschnitt gelöst werden, beispielsweise dadurch, dass der unelastische Abschnitt durchtrennt wird. Ist der unelastischen Abschnitt länger als der ungedehnte elastische Abschnitt, wirkt der unelastische Abschnitt als Längenbegrenzer und verhindert, dass der elastische Abschnitt mit einer Kraft beaufschlagt wird, die zu groß ist. Dadurch kann der elastische Abschnitt vor einer Schädigung bewahrt werden.

Wenn der unelastische Abschnitt reversibel an dem elastischen Abschnitt festgelegt ist, z.B. über Formschlusselemente in Gestalt von Haken, Ösen, Laschen oder einen Klettverschluss, ist es möglich die oben beschriebenen Effekte individuell zu nutzen. Bei einer Entfernung der unelastischen Abschnitte kommt es zu einer rein elastischen Ausgestaltung, da die unelastischen Abschnitte außer Funktion genommen wurden. Verändern sich dann die äußeren Gegebenheiten, beispielsweise in Verlauf eines sportlichen Wettkampfes oder bei nachlassender Kompression des Grundkörpers und des Stützgurtes, besteht die Möglichkeit, den elastischen Abschnitt mit dem unelastischen Abschnitt zu verstärken oder zu blockieren.

Die elastischen und unelastischen Abschnitte sind bevorzugt übereinander angeordnet und können einander zumindest teilweise überdecken. Beispielsweise kann der elastischen Abschnitt breiter als der unelastische sein und diesen aufnehmen.

Es ist möglich, dass mehrere parallel zueinander angeordnete elastische und unelastische Abschnitte in Reihe zueinander angeordnet sind, sodass eine Längenabstufung ohne weiteres möglich ist. Ebenfalls ist es möglich, die Gesamtdehnung einzustellen, indem einzelne unelastische Abschnitte außer Funktion gebracht werden, sodass die jeweils parallel dazu angeordneten elastischen Abschnitte leicht dehnbar sind.

Zwischen zwei elastischen Abschnitten kann ein unelastisches Gurtsegment angeordnet sein, sodass nicht der gesamte Stützgurt aus einem elastischen Grundabschnitt besteht, was jedoch auch möglich ist. Dann besteht der Stützgurt aus einem elastischen Grundabschnitt, auf dem ein unelastischer Abschnitt angeordnet ist.

Sofern ist vorgesehen ist, dass mehrere, parallel zueinander angeordnete elastische und unelastische Abschnitte in Reihe zueinander angeordnet sind, können die einzelnen Elastizitäten miteinander gekoppelt werden, wodurch eine große Variationsbreite zum Einstellen der Funktionalität des Gurtes bereitgestellt wird. Ist beispielsweise der unelastische Gurt kürzer als der ungedehnte elastische Gurt, kann durch das Durchtrennen mehrerer unelastischer Abschnitte die Elastizität stufenweise vergrößert werden. Bei einer reversiblen Festlegung kann durch das Aufheben der Verbindung eine vergrößere elastische Dehnbarkeit zur Verfügung gestellt werden, die durch erneutes Festlegen des unelastischen Abschnittes nach Belieben eingeschränkt werden kann.

Solange der unelastische Abschnitt kürzer als der ungedehnte elastische Abschnitt ist, ist der Stütz- und Fixiergurt vorzugsweise als ein Stabilisiergurt einsetzbar, der beispielsweise in der ersten Phase einer Traumatherapie stark stabilisierend verwendet wird. In der ersten Phase der Therapie kann es notwendig sein, das Gelenk oder die Gliedmaße zu stützen und ruhig zu stellen. Nach einer gewissen Zeit der Ruhigstellung ist im Rahmen einer frühfunktionellen Therapie eine schnellstmögliche Bewegung der jeweiligen Gliedmaße angezeigt. Dazu kann dann der kurze unelastische Abschnitt durchtrennt werden, sodass die Sperre, die der unelastische Abschnitt für den elastischen Abschnitt ausbildet, aufgehoben wird. Der elastische Abschnitt ist nun wirksam und kann eine Nachgiebigkeit des Stütz- und Fixiergurtes ermöglichen.

Ist dagegen der unelastische Abschnitt länger als der ungedehnte elastische Abschnitt ausgebildet, wirkt zunächst der elastische Abschnitt als eine Kraftübertragungseinrichtung. Wird die maximale Streckgrenze des elastischen Abschnittes erreicht, dient der unelastische Abschnitt als Überlastsicherung. Dazu ist es insbesondere vorgesehen, dass der unelastische Abschnitt zwar länger als der ungedehnte elastische Abschnitt ist, jedoch kürzer als die maximale Länge des elastischen Abschnittes, sodass der unelastische Abschnitt wirksam wird, bevor die maximale Dehnung des elastischen Abschnittes erreicht ist.

Eine Weiterbildung der Erfindung sieht vor, dass mehrere elastische Abschnitte parallel zueinander angeordnet sind, beispielsweise zwei oder mehr elastische Gurtabschnitte übereinander, um die Federkraft der elastischen Abschnitte zu vergrößern. Soll dann die Elastizität vergrößert werden, bei der bei einer gleichbleibenden Kraft eine größere Dehnung erreicht wird, ist es lediglich notwendig, einen oder mehrere elastische Abschnitte zu durchtrennen oder zu entfernen, um eine individuelle Einstellung der Elastizität und Funktionalität des Gurtes zu erreichen.

Die elastischen und unelastischen Abschnitte weisen bevorzugt die gleiche Breite auf, um einen Gurt mit einer durchgehend gleichen Breite bereitzustellen. Dazu ist es ggf. notwendig, dass auch das unelastische Gurtsegment oder die unelastischen Gurtsegmente die gleiche Breite wie die Abschnitte aufweisen. Alternativ ist vorgesehen, dass die unelastischen und elastischen Abschnitte unterschiedliche Breiten aufweisen, bevorzugt ist der unelastische Abschnitt schmaler als der elastische Abschnitt und auf diesem angeordnet.

Die Fixiereinrichtungen für den Stützgurt können als Flausch- und Hakenbereiche ausgebildet sein, wobei die Fixiereinrichtungen an den unelastischen Abschnitten oder an den unelastischen Gurtsegmenten angeordnet sein können. Auch wenn die elastischen und unelastischen Abschnitte nicht permanent miteinander verbunden sind, können sie unterschiedliche Längen aufweisen. Ebenfalls ist möglich, dass mehrere elastische Abschnitte parallel zueinander angeordnet sind, so dass durch eine Häufung der elastischen Abschnitte die aufzubringende Dehnungskraft variiert werden kann.

Der Grundkörper ist bevorzugt als eine Hülse ausgebildet, um über das dafür vorgesehene Gelenk oder die dafür vorgesehene Gliedmaße gezogen zu werden. Alternativ dazu kann Grundkörper auch flächig ausgebildet sein, versehen mit Verbindungselementen, um den Grundkörper um das Gelenk oder die Gliedmaße zu schließen. Die Verbindungselemente sind beispielsweise Klettverschlüsse, Ösen, Haken, Schnallen oder dergleichen.

Vorteilhafterweise ist die rutschhemmende Beschichtung nur bereichsweise auf der Innenseite des Grundkörpers angeordnet, um eine ständige Zuordnung des Grundkörpers zu der Gliedmaße des Bandagennutzers bereitstellen zu können. Bei einer vollflächigen Beschichtung besteht die Gefahr, dass die notwendige Durchlässigkeit für Feuchtigkeit nicht gegeben ist. Eine bereichsweise Beschichtung ist in der Regel ausreichend, um die Stützbandage an der Hautoberfläche sicher anzuordnen und ein unbeabsichtigtes Verdrehen oder Verschieben der Bandage auf der Hautoberfläche zu verhindern. Es ist möglich und vorgesehen, eine vollflächige, luftdurchlässige Beschichtung aufzubringen.

An der Außenseite des Grundkörpers können Bereiche mit Formschlusselementen zur Festlegung des Stützgurtes angeordnet sein, wobei die Formschlusselemente als Haken- oder Flauschbereiche eines Klettverschlusses ausgebildet sein können. Ebenfalls ist es möglich, dass andere Formschlusselemente vorhanden sind, beispielsweise Schlaufen, Laschen, Ösen, Druckknöpfe oder dergleichen. Die Bereiche sind vorzugsweise an physiologisch sinnvollen Stellen angeordnet, sodass eine entsprechende Verlegung des Stützgurtes nach dem Anlegen der Bandage erleichtert wird. Die Bereiche mit den Formschlusselementen können reversibel an der Außenseite des Grundkörpers angeordnet sein, damit diese individuell angepasst werden können.

Die Außenseite des Grundkörpers kann als Flauschschicht ausgebildet sein, wobei diese Flauschschicht vollständig die Außenseite des Grundkörpers bedecken kann. Die Formschlusselemente des Stützgurtes können als Hakenelemente ausgebildet sein, so dass der Stützgurt an jeder beliebigen Stelle der Außenseite des Grundkörpers festgelegt werden kann. Alternativ kann die Außenseite des Grundkörpers auch vollständig mit Hakenelementen überzogen sein, damit die Flauschbereiche des Stützgurtes an den zugewiesenen Stellen anhaften können. Der Grundkörper kann zumindest eine Ausnehmung aufweisen, um einen Bereich verminderter Druckwirkung bereitzustellen, beispielsweise um die Kniescheibe oder den Ellenbogen nicht mit einer Druckkraft zu belasten. Durch die Ausnehmung kann auch ein Körperteil hindurch gesteckt werden, beispielsweise der Daumen bei einer Handgelenksbandage, um ein Verrutschen zu verhindern.

Der Stützgurt kann einen Kraftindikator aufweisen, um den Nutzer der Stützbandage eine Rückmeldung und Orientierung zu geben, wie hoch die gegenwärtig aufgebrachte Kraft durch den Stützgurt und damit auf dem Grundkörper ist. Der Kraftindikator kann als ein sich in einem Fenster verschiebendes Element ausgebildet sein, beispielsweise an einem Fenster in dem unelastischen Abschnitt. Ebenfalls kann durch eine sich verändernde Farbgestaltung oder durch die Verlagerung einer Markierung die gegenwärtig aufgebrachte Dehnungskraft angezeigt werden. Der Kraftindikator ermöglicht es, bei einem Nachgeben des Grundkörpers dem Bandagennutzer die Information zu geben, dass der Stützgurt nachgespannt werden muss. Der Grundkörper kann eine Führung aufweisen, in der der Stützgurt eingeführt werden kann, um eine verschiebliche Lagerung innerhalb des Grundkörpers zu ermöglichen, ohne dass die Fixierwirkung dadurch beeinträchtigt wird. Diese Führung kann als Lasche, Schlitz oder dergleichen ausgebildet sein.

Neben einer bevorzugten geraden Ausführung des Stützgurtes ist es möglich, diesen in einer gebogenen Grundform auszubilden, um eine bessere Anpassung an die physiologischen Gegebenheiten des Bandagennutzers zu ermöglichen. So ist beispielsweise eine S-förmige Ausgestaltung des Stützgurtes günstig, um die Kraft um die Patella herum aufzubringen.

An dem Textilmaterial oder dem Stützgurt können elastische Elemente befestigt sein, die eine bereichsweise Änderung der Elastizität der Stützbandage bewirken, beispielsweise indem mehrere elastische Elemente miteinander gekoppelt werden oder einzelne elastische Elemente an speziellen Bereichen des Textilmaterials festgelegt werden, um eine Spannung aufzubringen. Das Textilmaterial kann elastisch ausgebildet sein. Die Elastizitäten des Grundkörpers oder des Stützgurtes lassen sich auch über Perforationen einstellen, die in dem jeweiligen Material eingebracht sind.

Das Material des Grundkörpers ist bevorzugt scherstabil ausgebildet, so dass die Oberseite nicht relativ zu der dem Körper zugewandten Unterseite verlagert werden kann. Die Scherkräfte wirken dabei parallel zu der Ebene des Grundkörpers. Die Scherstabilität wird dabei durch den Aufbau des Materials und die Dicke des Materials beeinflusst, je dicker das Material gewählt ist, desto geringer ist die Scherstabilität, da sich die Oberseite relativ zu der Unterseite leichter verlagern lässt.

Die Stützgurte können unterschiedliche Zugelastizitäten aufweisen, die durch unterschiedliche Materialien, Perforationen oder Herstellverfahren erzeugt werden können.

Der Stützgurt kann in einer weiteren Ausgestaltung zumindest teilweise auf der Innenseite des Grundkörpers angeordnet oder entlanggeführt sein, so dass der Stützgurt zumindest teilweise direkt auf der Hautoberfläche aufliegt und eine Stimulierung des darunter liegenden Muskels bewirkt. Die Lagestabilität wird beispielsweise durch eine auf der der Hautoberfläche zugewandten Innenseite des Stützgurtes vorgesehene rutschhemmende Beschichtung bewirkt, die durch die Lagestabilität des Grundkörpers auf der Gliedmaße unterstützt wird.

An dem Stützgurt können Noppen, Druckelemente, Vorsprünge, Kugelsegmente oder andere Einrichtungen angebracht sein, die lokal eine Druckerhöhung auf die beaufschlagte Muskelpartie oder den beaufschlagten Teil der Gliedmaße verursachen. Es können einzelne Einrichtungen oder Elemente ebenso wie mehrere Einrichtungen oder Elemente vorgesehen sein, insbesondere um die Propriozeption zu erhöhen. Die Einrichtungen zur lokalen Druckerhöhung können alternativ oder ergänzend an dem Grundkörper angeordnet sein, wo sie auf die vorgesehene Stelle einwirken. Eine Verstärkung des Druckes kann durch Anlegen und Spannen des Stützgurtes erfolgen, der über die Einrichtung gelegt und an dem Grundkörper festgelegt wird. Die Einrichtungen zur lokalen Druckerhöhung können an der Außenseite des Grundkörpers angeordnet sein, ebenso besteht die Möglichkeit, dass die Einrichtungen zur lokalen Druckerhöhung an der Innenseite des Stützgurtes und/oder des Grundkörpers angeordnet sind. Bei einer Anordnung auf der Innenseite kann ein unmittelbarer Kontakt zur Hautoberfläche hergestellt werden, ebenso ist eine gute Abschirmung und Abdeckung durch den Grundkörper und ggf. den Stützgurt gegeben. Eine Anordnung auf der Außenseite des Grundkörpers erleichtert die Positionierung an der richtigen Stelle. Die Einrichtungen zur lokalen Druckerhöhung können reversibel an dem Grundkörper und/oder dem Stützgurt festlegbar ausgestaltet sein, beispielsweise über einen Klettverschluss. Die Einrichtungen sind beispielsweise aus Kunststoff ausgebildet und bevorzugt abgerundet geformt, um einen angenehme Tragbarkeit zu erreichen.

In dem Grundkörper können Durchführungen für den Stützgurt angeordnet sein, so dass eine Festlegung des Enden oder auch von Zwischenbereichen des Stützgurtes auf der Oberseite des Grundkörpers erfolgen kann. Dadurch können die Formschlusselemente, über die der Stützgurt auf dem Grundkörper festgelegt werden kann, leicht zugänglich auf die Außenseite des Stützbandage verbleiben, während Teile des Stützgurtes auf der Innenseite in direktem Kontakt zu der Hautoberfläche stehen.

Die Stützbandage ist zur Verwendung als eine die Muskulatur unterstützende Bandage vorgesehen, die nicht zur Stabilisierung eines Gelenkes durch eine Einschränkung der Bewegung durch eine Ableitung von Kräften auf außenliegende Versteifungselemente vorgesehen ist. Vielmehr soll eine den Muskel unterstützende Funktion durch Ausübung von Druck auf den unterhalb des Stützgurtes liegenden Muskel vorgesehen. Dazu wird der Stützgurt im Wesentlichen entlang des Muskelverlaufes auf dem Grundkörper orientiert festgelegt, also entlang dem Muskel oder in einem spitzen Winkel diagonal zu der Längserstreckung des Muskels. Durch die reversible Anordnung des Stützgurtes auf dem Grundkörper kann eine wiederholte Nutzung erfolgen, insbesondere kann während der Nutzung eine Justierung und eine Erhöhung oder Verringerung der Spannung auf den Muskel durch eine einfache Verlagerung der Enden des Stützgurtes auf dem Grundkörper erfolgen. Die Stützgurte wirken somit als Kompressionselemente, die auf den jeweiligen Muskel wirken, und nicht als Befestigungselement, die durch zirkuläre Spannung eine Verlagerung einer Orthese auf der Gliedmaße oder eine Bewegungseinschränkung bewirken sollen. Vielmehr erfolgt eine partielle Druckbeaufschlagung des Muskels, ohne gewollte Behinderung der Blutzufuhr durch Anlegen elastischer Kompressionsgurte. Sowohl der Grundkörper als auch die Kompressionsgurte, also alle Komponente der Bandage, sind flexibel, so dass keine Gelenkversteifung oder Bewegungsbehinderung erfolgt, sollte eine Bandage über einem Gelenk getragen werden. Es ist auch vorgesehen, dass die Bandage nur so getragen wird, dass kein Gelenk überdeckt wird. Die lokal wirkende Kompression ist in Größe und Verlauf individuell einstellbar und beliebig oft nachjustierbar.

Nachfolgend werden Ausführungsbeispiele der Erfindung anhand der beigefügten Figuren näher erläutert. Es zeigen:
- Figur 1 -: eine erste Ausgestaltungsform der Stützbandage als Kniebandage;
- Figur 2 -: eine Innenseite der Kniebandage nach Figur 1;
- Figur 3 -: eine Stützbandage als Knöchelbandage;
- Figur 4 -: einen Stützgurt in Einzeldarstellung;
- Figur 5 -: eine Variante des Stützgurtes mit einem kurzen elastischen Abschnitt;
- Figur 6 -: eine Variante der Erfindung mit einem kurzen unelastischen Abschnitt;
- Figur 7 -: eine Detaildarstellung mit mehreren, parallel geschalteten elastischen Abschnitten;
- Figur 8 -: eine Variante der Figur 3;
- Figur 9 -: eine Variante der Figur 1;
- Figur 10 -: eine Detaildarstellung eines Stützgurtes;
- Figur 11 -: eine Variante des Stützgurtes;
- Figur 12 -: eine Variante der Bandage mit innenliegendem Stützgurt;
- Figur 13 -: eine Schrägdraufsicht der Figur 12; sowie
- Figur 14 -: eine Variante der Figur 13.

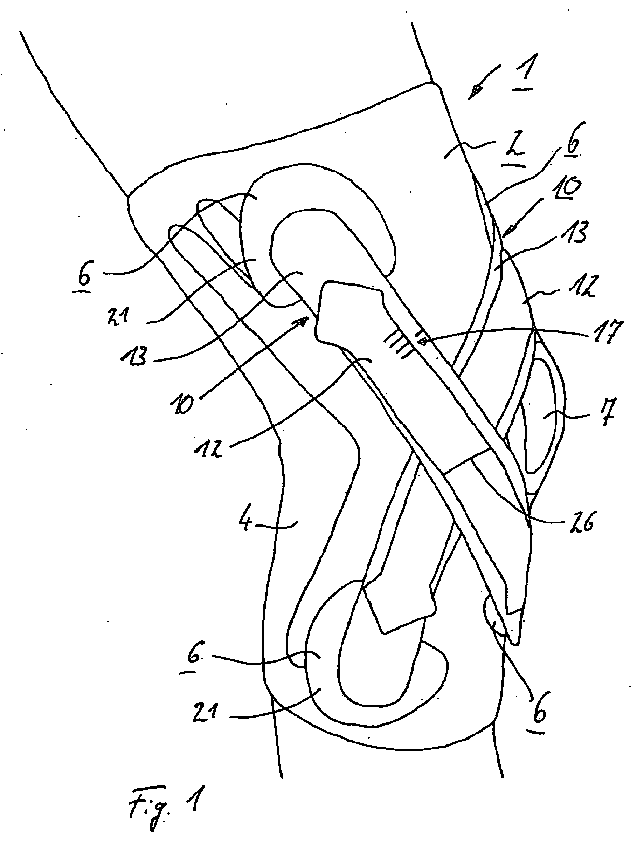

In der Figur 1 ist eine Stützbandage 1 mit einem Grundkörper 2 aus einem elastischen Textilmaterial gezeigt. Die Stützbandage 1, die als eine Kniebandage ausgebildet ist, ist in einem angelegten Zustand dargestellt, so dass nur die Außenseite 4 zu sehen ist. Im Bereich der Kniescheibe ist eine Ausnehmung 7 angeordnet, um unerwünschten Druck auf die Kniescheibe zu vermeiden. Die Außenseite 4 des Grundkörpers 2 weist gesondert markierte Bereiche 6 mit Formschlusselementen 21, beispielsweise in Gestalt von Flausch- oder Hakeneinrichtungen eines Klettverschlusses, auf. Diese Bereiche 6 mit Formschlusselementen 21 können an physiologisch für sinnvoll erachteten Stellen oder Bereichen angeordnet sein, wobei sich je nach Indikation die Lage und/oder Größe der Bereiche 6 verändern kann. Es ist ebenfalls möglich, dass eine Vielzahl solcher Bereiche 6 an der Außenseite 4 des Grundkörpers 2 angeordnet sind, die beispielsweise eine unterschiedliche farbliche Markierung aufweisen, um eine korrekte Zuordnung für den Stützgurt 10 zu ermöglichen, was nachfolgend genauer beschrieben werden wird. Auch ist es möglich und vorgesehen, dass die gesamte Außenseite 4 des Grundkörpers 2 als eine Flauschschicht ausgebildet ist, auf der die Bereiche 6 optisch abgegrenzt und markiert sind.

An der Außenseite 4 des Grundkörpers 2 der Stützbandage 1 sind zwei Stützgurte 10 festgelegt. Die Festlegung erfolgt über Formschlusselemente 16, die später näher erläutert werden. Die Formschlusselemente 16 können beispielsweise als Hakenelemente ausgebildet sein, über die die Enden der Stützgurte 10 in den Bereichen 6 mit der Flauschschicht 21 in Verbindung gebracht und an dem Grundkörper 2 festgelegt werden können. In der dargestellten Ausführungsform sind die Stützgurte 10 einander überkreuzend an der Außenseite 4 des Grundkörpers 2 angeordnet, wobei der Kreuzungspunkt seitlich neben der Patella liegt. Dadurch ist es möglich, eine zusätzliche Abstützung des Kniegelenkes und Führung der Patella zu ermöglichen.

Die Stützgurte 10 bestehen aus einem elastischen Abschnitt 13 und einem oberhalb darauf angeordneten, unelastischen, flexiblen Abschnitt 12. Der unelastischen Abschnitt 12 ist an dem elastischen Abschnitt 13, der die gesamte Länge des Gurtes ausmachen kann, über eine Fixiernaht 26 befestigt. Zum Anlegen des Stützgurtes 10 müssen die Enden lediglich in den Bereichen 6 mit der Flauschschicht 21 aufgelegt werden. Aufgrund der Elastizität des elastischen Abschnittes 13 ist es möglich, eine Zugkraft aufzubringen, die zwischen den Befestigungsbereichen 6 wirkt und bei einer Kniebandage zu einer Stabilisierung des Knies führt. Über den flexiblen, unelastischen Abschnitt 12 ist es möglich, statt einer elastischen Zugkraft eine mehr oder weniger starre Kompression aufzubringen, um die Stützwirkung zu erhöhen.

An dem unelastischen Abschnitt 12 sind mehrere Markierungen in Gestalt von Strichen angeordnet, über die es möglich ist, einen Kraftindikator auszubilden. An dem elastischen Abschnitt 13 ist ebenfalls eine Markierung angeordnet, die je nach Aufbringung der Zugkraft einer entsprechenden Markierung auf dem unelastischen Abschnitt 12 gegenüber liegt. Je weiter die Markierung des elastischen Abschnittes 13 von der Fixiernaht 26 entfernt ist, desto größer ist die aufgebrachte Zugkraft. Andere Kraftindikatoren 17 sind vorgesehen und möglich, beispielsweise Fenster, Schlaufen, die Anordnung mehrerer Markierungen auf dem elastischen Abschnitt 13 bei einer einzigen Referenzmarkierung auf dem unelastischen Abschnitt 12 oder dergleichen.

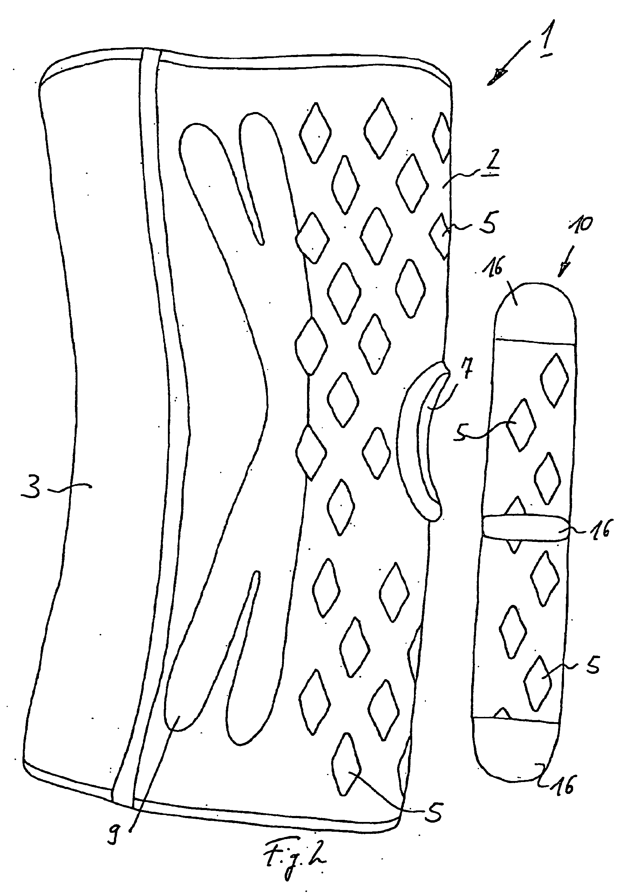

In der Figur 2 ist die Stützbandage 1 gemäß Figur 1 mit der Innenseite 3 nach außen dargestellt. Es ist zu erkennen, dass auf der Innenseite 3 der Stützbandage 1 auf dem Grundkörper 2 rutschhemmende Beschichtungspunkte 5 angeordnet sind, die vermeiden, dass die Stützbandage 1 im angelegten Zustand auf der Hautoberfläche verrutscht. Die rutschhemmende Beschichtung 5 ist bevorzugt aus einem Silikonmaterial ausgebildet, andere Materialien wie z.B. PU können ebenfalls vorgesehen sein. Neben einer punktuellen oder bereichsweisen Anordnung der rutschhemmenden Beschichtung 5 können auch großflächige Bereiche mit der rutschhemmenden Beschichtung 5 vorgesehen sein.

Ebenfalls ist in der Figur 2 zu erkennen, dass eine Stabilisierungsschiene 9 zur Erhöhung der seitlichen Stabilität in dem Grundkörper 2 eingearbeitet ist. Die Stabilisierungsschiene 9 ist aus einem formstabilen Material ausgebildet, vorzugsweise Kunststoff, und lässt eine gewisse Flexibilität in medialer und lateraler Richtung zu.

In der Figur 2 ist ebenfalls die Unterseite des Stützgurtes 10 dargestellt. Der Stützgurt 10 kann ebenfalls eine rutschhemmende Beschichtung 5 aufweisen, was vorteilhaft ist, wenn der Stützgurt 10 unmittelbar auf der Haut getragen werden soll. Darüber hinaus ist zu erkennen, dass Formschlusselemente 16 sowohl an den oberen und unteren Enden als auch in der Mitte des Stützgurtes 10 angeordnet sind. Über diese Formschlusselemente 16, insbesondere Hakenbereiche, ist es möglich, den Stützgurt 10 fest und reversibel auf der Außenseite 4 des Grundkörpers 2 der Stützbandage 1 zu befestigen.

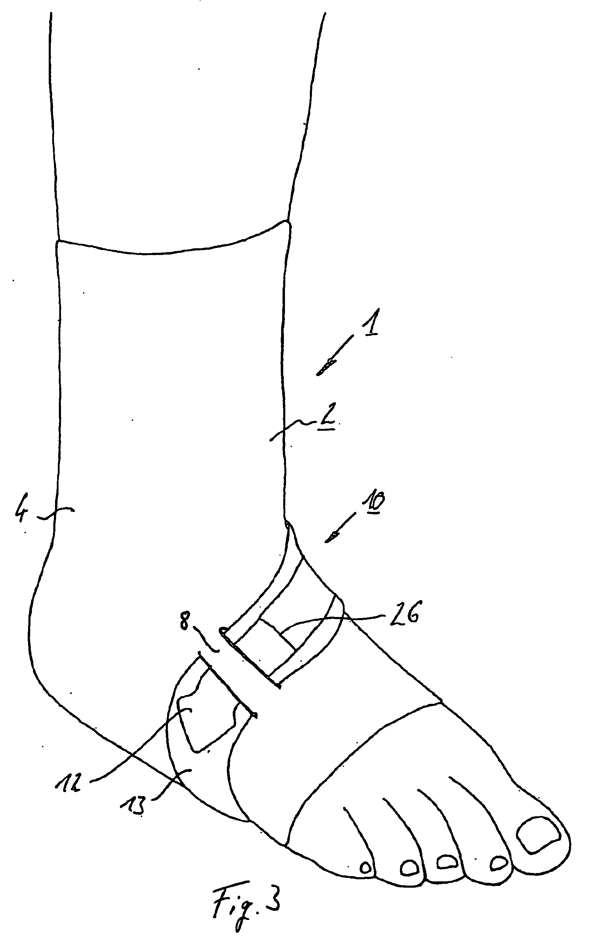

In der Figur 3 ist eine Knöchelbandage 1 mit einem Grundkörper 2 gezeigt, bei der innerhalb des Grundkörpers 2 eine Lasche 8 als Führung für den Stützgurt 10 ausgebildet ist. Neben einer einstückigen Ausbildung der Führung 8 kann diese auch aufgenäht sein. Andere Führungselemente oder Einrichtungen können ebenfalls vorgesehen sein, um eine sichere Zuordnung des Stützgurtes 10 auf der Bandage und damit zu der Gliedmaße, um die herum die Stützbandage 1 angelegt ist, zu ermöglichen. Auch hier sind elastische und unelastische Abschnitte 13, 12 übereinander angeordnet. Der unelastische Abschnitt 12 ist über die Fixiernaht 26 an dem elastischen, unterhalb des unelastischen Abschnittes 12 angeordneten elastischen Abschnitt 13 festgelegt. Über die Lage der Enden des elastischen Abschnittes 13 ist es möglich, die jeweils aufgebrachte Zugkraft und damit die Stabilisierungswirkung des Stütz- und Fixiergurtes 10 einzustellen. Auch ist es möglich, durch die reversible Festlegung des Stütz- und Fixiergurtes 10 eine Nachjustierung sowie eine stufenlose Einstellung der auf die Stützeinrichtung 1 aufgebrachten Kräfte zu erreichen. Durch die Anordnung von Stütz- und Fixiergurten 10 auf dem Grundkörper kann ein sehr differenzierter Kräfteaufbau in der Stützeinrichtung erzeugt werden. Die Stütz- und Fixiergurte 10 bewirken eine Zugerhöhung lediglich in Teilbereichen der Stützbandage 1, so dass gezielt Kräfte auf die Gliedmaße aufgebracht werden können. Die Übertragung der durch die elastischen Abschnitte 13 erfolgt dabei über die rutschhemmende Beschichtung 5, die als Interface zwischen dem textilen Grundkörper 2 und der Haut wirkt. Die unterschiedlichen Zug- bzw. Querkräfte werden nicht durch eine aufwendige Fertigungstechnik für den Grundkörper 2 erreicht, sondern können einfach und individuell verwirklicht werden, ohne dass eine spezielle Stützbandage 1 angefertigt werden müsste. Die Formschlusselemente 21 auf der Außenseite 4 der Stützbandage 1 können elastisch als Interface ausgebildet sein, sodass ein breiter Einsatzbereich des Grundkörpers 2 gegeben ist. Es können vorgegebene Bereiche oder Zonen zum Festlegen der Stützgurte 1 auf der Außenseite 4 der Stützbandage 1 markiert sein, um das Gelenk korrekt zu unterstützen. Eine Nachjustierung der Spannung kann durch einfaches Lösen und Verschieben und erneutes Festlegen der Enden des Stützgurtes 10 erfolgen.

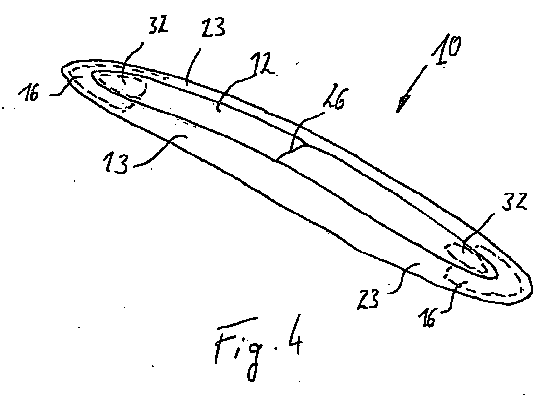

In der Figur 4 ist in einer prinzipiellen Darstellung ein Fixier- oder Stützgurt 10 gezeigt, der in der dargestellten Ausführungsform einen durchgehenden elastischen Gurtabschnitt 13 aufweist, der auf seiner Oberseite 23 mit einer Flauschschicht ausgestattet sein kann. An der Unterseite sind Fixiereinrichtungen 16 zum Festlegen der Endbereiche aneinander oder an einer orthopädietechnischen Einrichtung, insbesondere der Stützbandage 1 vorgesehen. Auch diese Fixiereinrichtungen 16 können als Flauschbereiche oder als Hakenbereiche eines so genannten Klettverschlusses ausgebildet sein. Alternative Fixiereinrichtungen 16, beispielsweise Haken, Druckknöpfe, Ösen oder dergleichen, können ebenfalls vorgesehen sein.

Auf der Oberseite 23 des elastischen Abschnittes 13 ist ein unelastischer Abschnitt 12 angeordnet, der im dargestellten Ausführungsbeispiel als ein langgestrecktes Gurtelement ausgebildet ist. Die Fixierung des unelastischen Abschnittes 12 an dem elastischen Abschnitt 13 erfolgt über eine Naht 26, die im vorliegenden Ausführungsbeispiel zentral angeordnet ist. Andere Befestigungseinrichtungen können ebenfalls vorgesehen sein, beispielsweise kann der unelastische Abschnitt 12 an dem elastischen Abschnitt 13 angeschweißt oder angeklebt sein. Grundsätzlich ist auch eine reversible Fixierung mittels Klettverschlüssen oder dergleichen möglich.

An der Unterseite des unelastischen Abschnittes 12, also an derjenigen Seite, die der Oberseite 23 des elastischen Abschnittes 13 zugewandt ist, sind Formschlusselemente 32 in Gestalt von Hakenbereichen eines Klettverschlusses ausgebildet. Über diese Formschlusselemente 32 ist es möglich, den unelastischen Abschnitt 12 an nahezu beliebiger Stelle zwischen der Maximalerstreckung des unelastischen Abschnittes 12 und der Befestigungsnaht 26 festzulegen. Auch hier sind der unelastische Abschnitt 12 und der elastische Abschnitt 13 des Gurtes 10 parallel zueinander angeordnet. Durch die reversible Festlegung des unelastischen Abschnittes 12 auf den elastischen Abschnitt 13 über die Klettverschlüsse 32 ist es möglich, in unterschiedlicher Entfernung von einer, ggf. permanenten, Fixierstelle 26 eine Dehnungsbegrenzung zu installieren. Je weiter entfernt das jeweilige Ende des unelastischen Abschnittes 12 von der Befestigungsstelle 26 an dem elastischen Abschnitt 13 festgelegt wird, desto geringer ist die mögliche Dehnung des elastischen Abschnittes 13. Wird die Festlegung so gewählt, dass der elastische Abschnitt 13 im ungedehnten Zustand, also ohne dass durch Aufbringen einer Kraft eine elastische Verformung auftritt, länger ist als der unelastische Abschnitt 12, wird der durch den unelastischen Abschnitt 12 überbrückte Bereich seiner elastischen Wirkung beraubt. Erstreckt sich dann der unelastische Abschnitt 12 von dem einen äußeren Ende bis zum anderen äußeren Ende des Gurtes 10, ist eine Elastizität nicht mehr gegeben, erst nach Auflösen der Verbindung an den Formschlusselementen 32 wird dann eine elastische Komponente wieder bereitgestellt.

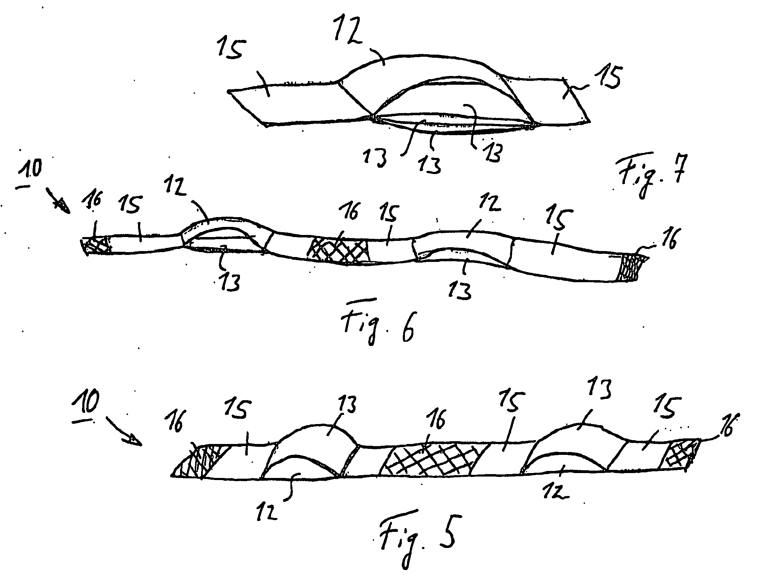

In der Figur 5 ist ein Stütz- oder Fixiergurt 10 gezeigt, der langgestreckt ausgebildet ist. Der Stütz- oder Fixiergurt 1 weist eine gleichmäßige Breite auf und ist in der Figur 5 flach liegend dargestellt. Der Stütz- oder Fixiergurt 10 weist an seinen Enden Fixiereinrichtungen 16 auf, mit denen die Gurtenden aneinander oder an der Außenseite 4 des Grundkörpers 2 der Stützbandage 1 festgelegt werden können. In der Mitte, in einem unelastischen, flexiblen Gurtsegment 15, ist ebenfalls eine Fixiereinrichtung 16 angeordnet, die beispielsweise als ein Flausch- oder Hakenbereich eines so genannten Klettverschlusses ausgebildet sein kann. An die Gurtenden mit den Fixiereinrichtungen 16 schließen sich ebenfalls unelastische Gurtsegmente 15 an. Zwischen den unelastischen Gurtsegmenten 15 sind parallel geschaltete, elastische und unelastische Abschnitte 13, 12 angeordnet. In dem dargestellten Ausführungsbeispiel ist der unelastische Abschnitt 12 kürzer als der elastische Abschnitt 13 in dem ungedehnten Zustand. Der Stütz- oder Fixiergurt 10 gemäß Figur 5 liegt somit in der maximal gestreckten Länge vor und weist schlaufenartige, elastische Abschnitte 13 auf. Wird der Stütz- oder Fixiergurt 10 gemäß Figur 5 angelegt, ist dieser zunächst starr. Der Stütz- oder Fixiergurt 10 kann auch unmittelbar an einem Körperteil angeordnet werden, um beispielsweise ein Gelenk stabilisieren und ruhig zu stellen. Ebenfalls ist es möglich, diesen Stütz- oder Fixiergurt 10 an eine Prothese oder Orthese anzulegen oder, wie beschrieben, dass er eine Manschette oder Bandage unterstützt. Dazu kann der Stütz- oder Fixiergurt 10 über die Fixiereinrichtungen 16 auch an der jeweiligen orthopädietechnischen Einrichtung angelegt und daran befestigt werden.

Nach der Stabilisierung des Gelenkes oder der orthopädietechnischen Einrichtung ist es möglich, durch Durchtrennen der unelastischen Abschnitte 12 oder eines unelastischen Abschnitte 12 eine gewisse Elastizität bereitzustellen, die durch die elastischen Abschnitte 13 bzw. den entsperrten und wirksam gemachten elastischen Abschnitt 13 bereitzustellen. In dem dargestellten Ausführungsbeispiel sind zwei Bereiche mit parallel geschalteten elastischen und unelastischen Abschnitten 13, 12 vorgesehen, es besteht jedoch auch die Möglichkeit, mehr als zwei solcher Abschnitte in einem Gurt 10 anzuordnen.

Eine Variante des Stützgurtes 10 ist in der Figur 6 gezeigt, bei der die gleichen Bezugszeichen die gleichen Komponenten bezeichnen. Anders als in der Figur 5 ist der elastische Abschnitt 13 im ungedehnten Zustand kürzer als der unelastische Abschnitt 12, so dass von Anfang an eine gewisse Elastizität in dem Stütz- und Fixiergurt 10 vorhanden ist. Die Länge des unelastischen Abschnittes 12, die im dargestellten, unelastischen Zustand größer als die Länge des ungedehnten elastischen Abschnittes 13 ist, ist vorzugsweise so gewählt, dass der elastische Abschnitt 13 nicht überdehnt wird oder über den vorgesehenen Dehnbereich hinaus nicht gestreckt werden kann. Dadurch wird eine übermäßige Belastung des elastischen Bereiches 13 vermieden, wodurch eine vergrößerte Dauerhaltbarkeit gewährleistet wird, da der elastische Abschnitt 13 nicht über die maximale Dehngrenze hinaus gestreckt wird.

Neben den dargestellten Ausführungsbeispielen ist es möglich, die unterschiedlichen Längengestaltungen der elastischen und unelastischen Abschnitte 12, 13 zu kombinieren, sodass ein oder mehrere parallel geschaltete elastische und unelastische Abschnitte 12, 13 gemäß der Figur 5 mit den elastischen und unelastischen Abschnitten 12, 13 gemäß Figur 6 kombiniert werden können. Dadurch ist es möglich, durch das Durchtrennen der "kurzen" unelastischen Abschnitte 12 eine vergrößerte Elastizität des Stütz- oder Fixiergurtes 10 bereitzustellen, wenn dieser bereits eine Grundelastizität aufweist, die durch die unelastischen Abschnitte 12 abgesichert ist.

Eine besondere Ausgestaltung des Stützgurtes ist in der Figur 7 dargestellt, in der gezeigt ist, dass eine Vielzahl an elastischen Gurtabschnitte 13, vorliegend drei elastischen Gurtabschnitten, parallel zueinander geschaltet angeordnet sein können. Über diese Vielzahl der elastischen Abschnitte 13 ist es möglich, einen größeren elastischen Widerstand bereitzustellen. Durch Entfernen, Durchtrennen oder dergleichen ist es möglich, diesen elastischen Widerstand einzustellen.

Die elastischen Abschnitte 13 können an dem Gurtbandmaterial angenäht, angeklebt oder angeschweißt sein. Die unelastischen Abschnitte 12 können aus dem gleichen oder demselben Material wie die unelastischen Gurtsegmente 15 ausgebildet sein. Es wird dann ein durchgängiger, einstückiger Gurt aus unelastischen Abschnitten 12 und unelastischen Gurtsegmenten 15 ausgebildet. Auch ist es möglich, dass die Fixiereinrichtungen 16 aufgenäht, aufgeklebt oder aufgeschweißt sind. Alternativ können diese Fixiereinrichtungen 16 an Enden des Gurtbandes 1 angenäht sein, sodass sie an den jeweiligen Enden der unelastischen Gurtabschnitte 12 bzw. unelastischen Gurtsegmente 15 angefügt sind.

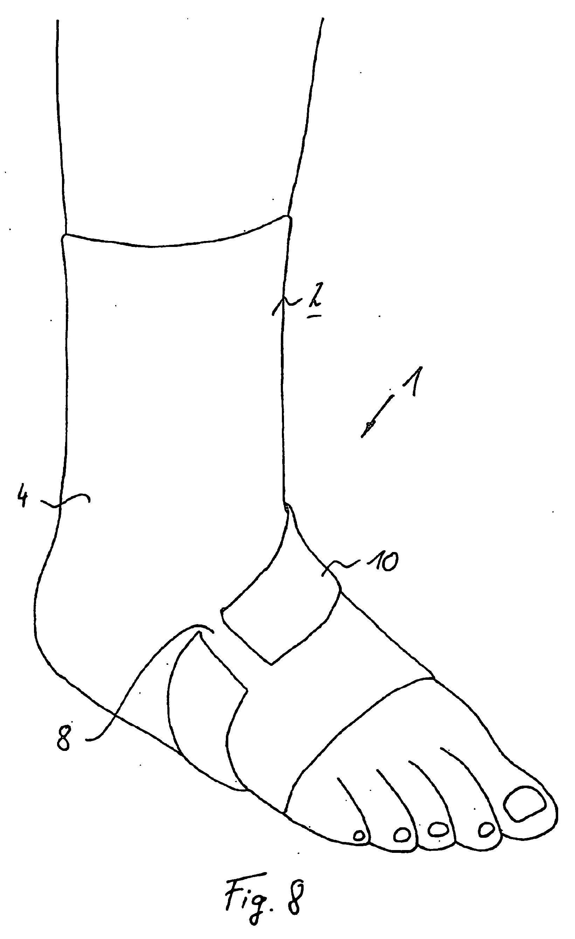

In der Figur 8 ist eine Variante der Erfindung gezeigt, die im Wesentlichen der Ausführungsform gemäß der Figur 3 entspricht. Statt eines mehrteilig ausgebildeten Stützgurtes 10, der gemäß Figur 3 einen unelastischen Abschnitt 12 und einen elastischen Abschnitt 13 aufweist, sieht der Stützgurt 10 gemäß der Figur 8 einen elastischen Abschnitt vor, so dass der gesamte Stützgurt 10 elastisch verformbar ist. Die elastischen Stützgurte 10 können wie in den vorbeschriebenen Ausführungsformen an Bereichen mit Formschlusselementen angeordnet werden, wobei diese Bereiche auch reversibel an der Außenseite 4 des Grundkörpers 2 angeordnet werden können. Die Innenseite des Grundkörpers 2 kann punktuell oder vollflächig beschichtet werden, beispielsweise mit einem Polyurethan, einem Silikon oder einem ähnlichen, rutschhemmenden Werkstoff. Der Grundkörper 2 kann, wie auch in den vorbeschriebenen Ausführungsformen, aus einem scherstabilen Material ausgebildet sein, das eine Relativverlagerung der Oberseite zur Unterseite bzw. der Außenseite zu der Innenseite nicht oder nur in einem vernachlässigbar geringen Umfang zulässt.

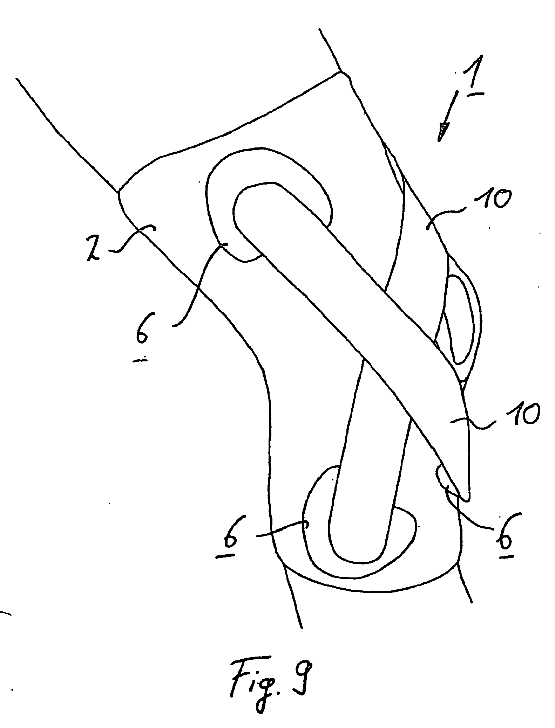

In der Figur 9 ist eine Variante der Erfindung gemäß Figur 1 dargestellt und zeigt, dass eine Kniebandage 1 mit einem schlauchartigen Grundkörper 2, an dem Bereiche 6 mit Formschlusselementen zur Festlegung der Stützgurte 10, die vorliegend kreuzweise geführt sind, angeordnet sind. Die Bereiche 6 sind beispielsweise als Flauschbereiche ausgebildet, die Teil eines Klettverschlusses in Wechselwirkung mit Hakenelementen an den Spanngurten 10 sind. Die Spanngurte 10 sind im vorliegenden Ausführungsbeispiel ebenfalls als rein elastische Spanngurte 10 ausgebildet, die beispielsweise aus einem elastischen Kunststoff, einem Gummimaterial, einem elastischen Gewebe oder dergleichen bestehen können. Die Bereiche 6 können über Klettvelours an physiologisch geeignete Stellen an dem Grundkörper 2 befestigt werden. Das Material des Grundkörpers 2 ist sehr dünn und weist eine Stärke von unter einem Millimeter auf, wodurch sich eine hohe Elastizität ergibt. Aufgrund des geringen Abstandes der Stützgurte 10 zu der Hautoberfläche werden die durch die Stützgurte 10 ausgeübten Zug- und Druckkräfte direkt auf die Haut und die darunter liegende Extremität übertragen. Die Stützgurte 10 sind in unterschiedlichen Elastizitäten anlegbar, so dass eine entsprechend angepasste Zugkraft und Spannung auf den Körperteil ausgeübt werden kann.

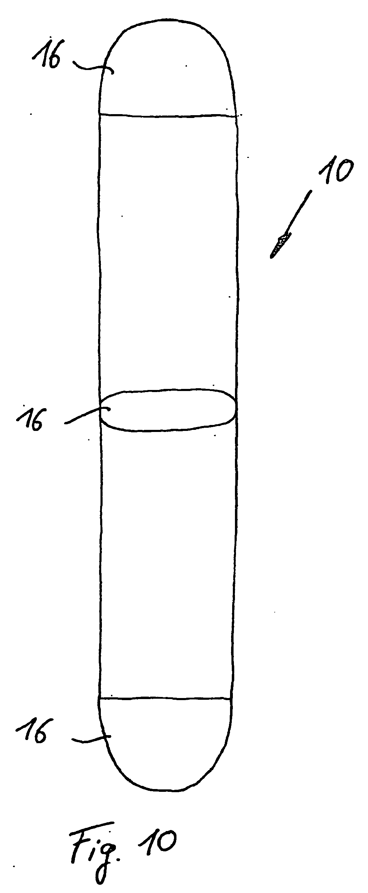

In der Figur 10 ist eine Variante des Stützgurtes 10 dargestellt, bei der auch hier eine rein elastische Ausgestaltung des Stützgurtes 10 vorgesehen ist. An verschiedenen Bereichen des Stützgurtes 10 sind auf der Unterseite Fixiereinrichtungen 16 angebracht, beispielsweise angenäht, angeklebt oder angeschweißt. Im dargestellten Ausführungsbeispiel sind die Fixiereinrichtungen 16 in Gestalt von Hakenelementen eines Klettverschlusses an den beiden Enden sowie in der Mitte des Stützgurtes 10 angeordnet, um auch eine winkelige Anordnung des Stützgutes 10 an dem Grundkörper zu gewährleisten.

Neben den dargestellten Ausführungsbeispielen können die Grundkörper auch abweichende Ausgestaltungen aufweisen, beispielsweise in einer Hosenform oder Hemdform ausgebildet sein, um Unterstützungen an den jeweils gewünschten Stellen des Körpers bereitstellen zu können.

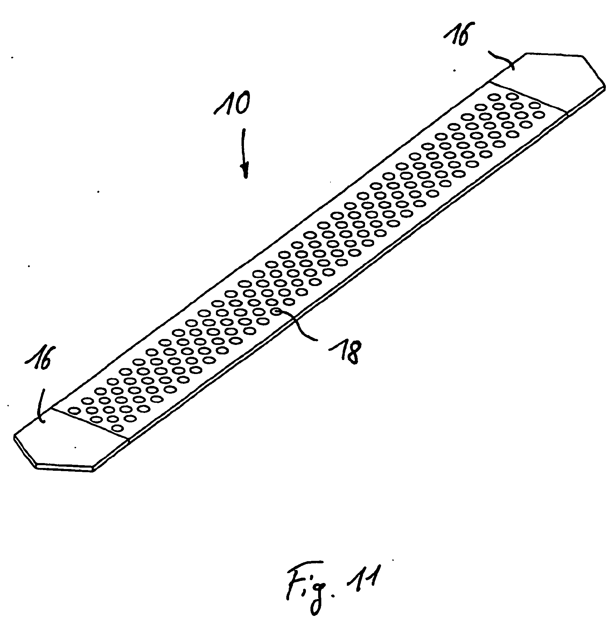

In der Figur 11 ist eine Detaildarstellung eines Stützgurtes 10 dargestellt, der auch als ein lokales Kompressionselement bezeichnet werden kann, da dieser Stützgurt 10, ebenso wie die Stützgurte in den vorhergehenden Figuren, eine lokale Kompression auf das darunter liegende Muskelgewebe bewirkt, so dass eine Propriozeption des Muskels erreicht wird. An den Enden des Stützgurtes 10 sind Formschlusselemente 16 vorgesehen, über die der Stützgurt 10 an einem Grundkörper festgelegt werden kann. Zwischen den Formschlusselementen 16 sind Perforationen 18 eingebracht, um die Zugelastizität insbesondere in Längserstreckung des Stützgurtes 10 einstellen zu können. Auf der Unterseite des Stützgurtes 10 können rutschhemmende Beschichtungen aufgebracht sein, wie sie beispielsweise in der Figur 2 dargestellt sind. Dadurch ist es möglich, dass es bei einer direkten Auflage des Stützgurtes 10 auf der Haut nicht zu einem ungewollten Verrutschen kommt. Die Formschlusselemente 16 sind entweder auf der Hautoberfläche abgewandten oder zugewandten Seite des Stützgurtes 10 angeordnet, je nachdem, wie der Stützgurt 10 an dem Grundkörper befestigt ist. Bei einer rein außenliegenden Befestigung sind die Formschlusselemente, beispielsweise in Gestalt von Klettverschlüssen, Druckknöpfen oder dergleichen, auf der Unterseite angeordnet, also derjenigen Seite, die dem Körper zugewandt ist. Eine gleiche Anordnung liegt vor, wenn der Stützgurt 10 innenliegend geführt ist, also dass Bereiche des Stützgurtes unmittelbar auf der Haut aufliegen oder in einer Führung innerhalb des Grundkörpers 2 geführt werden und die Festlegung des Stützgurtes 10 auf der Außenseite des Grundkörpers 2 erfolgt. Sofern eine vollständige Befestigung auf der Innenseite des Grundkörpers vorgesehen ist, sind die Formschlusselemente 16 auf der der Körperoberfläche abgewandten Seite des Stützgurtes 10 angeordnet. Die Orientierungen beziehen sich jeweils auf eine bestimmungsgemäß angelegte Stützbandage.

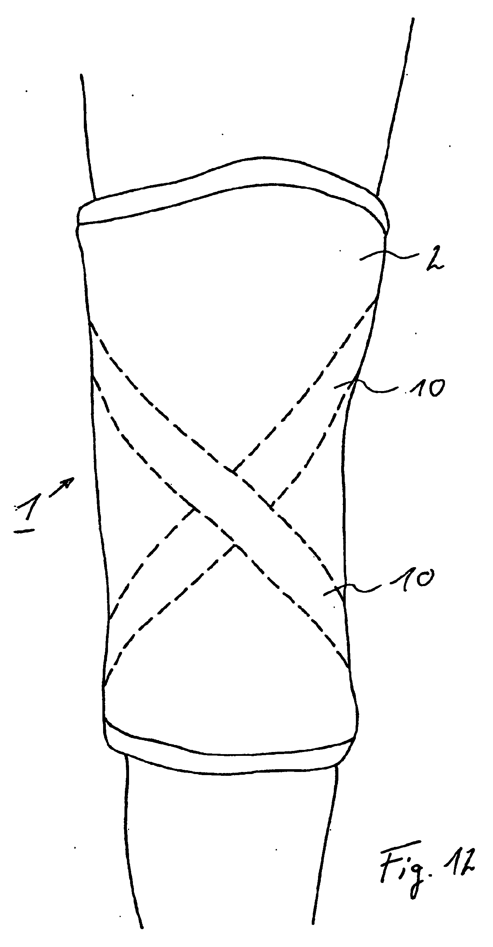

Die Figur 12 zeigt eine angelegte Stützbandage 1 mit einem Grundkörper 2 und innenliegenden Stützgurten 10, die nach außen von dem Grundkörper 10 abgedeckt sind. Die Stützgurte 10 sind in dem dargestellten Ausführungsbeispiel sich kreuzend geführt, um eine muskelaktivierende Kompression im Kniekehlenbereich einer Kniebandage zu bewirken.

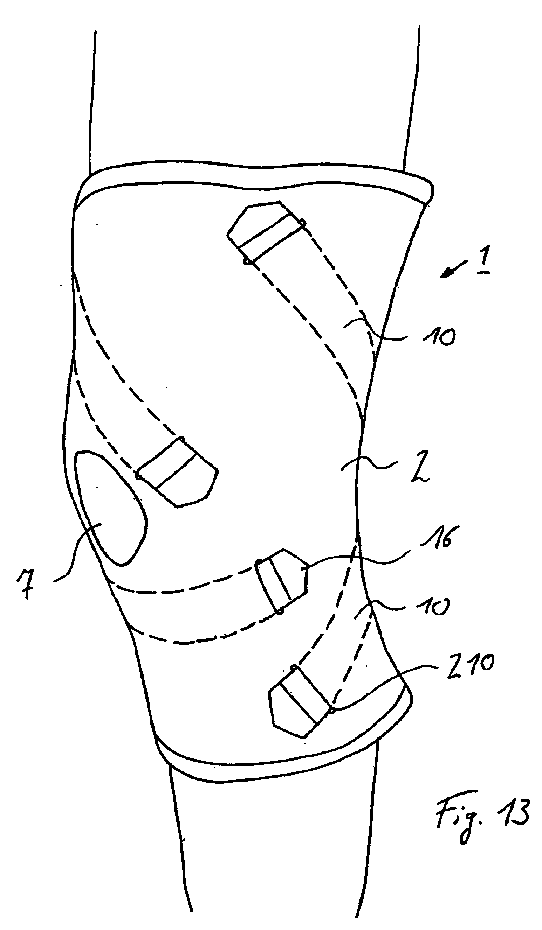

In der Figur 13 ist eine andere Ansicht der Ausführungsform gemäß Figur 12 dargestellt, nämlich eine Schrägdraufsicht von vorn. Es ist zu erkennen, dass in dem Grundkörper 2 Durchführungen 210 in Gestalt von Schlitzen ausgebildet sind, durch die die Enden der Stützgurte 10 hindurchgeführt werden. An diesen Enden sind die Formschlusselemente angeordnet, so dass nach dem Durchführen der Stützgurte 10 durch die Durchführungen 210 auf der Außenseite des Grundkörpers 2 eine einfache Fixierung der Stützgurte 10 vorgenommen werden kann. Bei nachlassender Kompressionswirkung, beispielsweise durch Erwärmung bei sportlicher Betätigung oder bei einer Umfangsgverringerung aufgrund von Abschwellvorgängen, kann die Spannung der Stützgurte 10 durch einfaches Lösen der reversibel auf der Oberfläche des Grundkörpers 2 festgelegten Formschlusselemente 16 und Straffen der vorzugsweise elastischen Stützgurte 10 justiert werden. Ebenfalls ist es möglich, bei einem zu hohen Spannungsgefühl die Kompressionswirkung zu verringern, indem die Formschlusselemente 16 von der Oberfläche oder den korrespondierenden Formschlusselementen des Grundkörpers 2 gelöst und näher in Richtung der Durchführung 210 erneut auf dem Grundkörper 2 festgelegt werden.

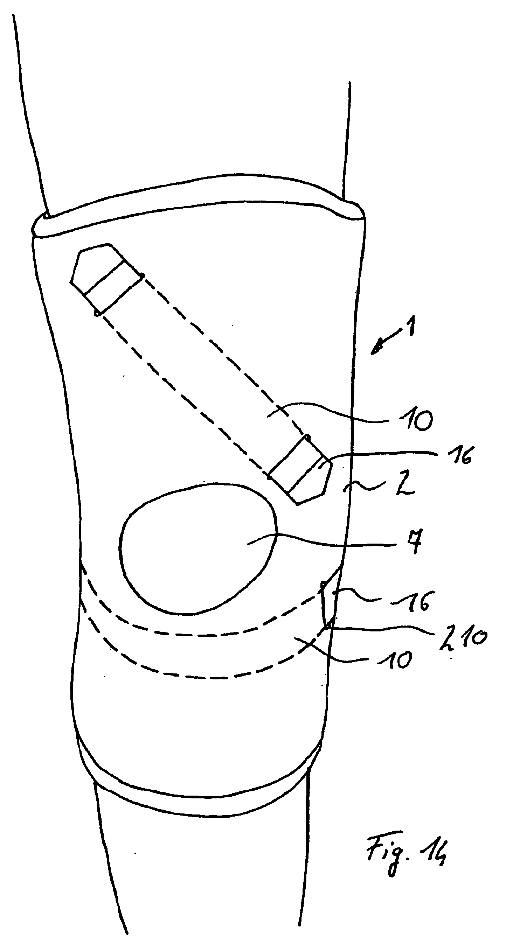

Eine Variante der Erfindung ist in der Figur 14 gezeigt, bei der die innenliegend geführten Stützgurte 10 einen anderen Verlauf als in der Figur 13 aufweisen. Der frontale Stützgurt 10 verläuft im Wesentlichen parallel zum Muskelverlauf mit einer leichten Winkelstellung dazu, der untere Stützgurt wirkt auf die Patellasehne, um eine Vorspannung zu erreichen.

Die Stützbandagen 1 dienen insbesondere zur Stimulierung von Muskeln oder Muskelvorspannungen, um gegebenenfalls vorhandene Dysbalancen auszugleichen. Eine Bewegungseinschränkung ist in der Regel nicht beabsichtigt, insbesondere bei Stützbandagen 1, die ausschließlich aus in allen Richtungen flexiblen Komponenten bestehen, ist eine Bewegungseinschränkung nicht möglich und vorgesehen.

## Patentansprüche

1. Stützbandage mit einem Grundkörper (2) aus einem elastischen Textilmaterial, der eine dem Körper eines Bandagennutzers zugewandte Innenseite (3) und eine dem Körper des Bandagennutzers abgewandte Außenseite (4) aufweist, mit einer auf der Innenseite (3) angeordneten rutschhemmenden Beschichtung (5), **dadurch gekennzeichnet, dass** die Stützbandage (1) zumindest einen Stützgurt (10) aufweist, der auf der Außenseite (4) des Grundköpers (2) über Formschlusselemente (21) reversibel angeordnet ist, und dass auf der der Hautoberfläche zugewandten Innenseite der Stützgurt (10) eine rutschhemmende Beschichtung (5) aufweist.

2. Stützbandage nach Anspruch 1, **dadurch gekennzeichnet, dass** der Stützgurt (10) zumindest einen flexiblen, unelastischen Abschnitt (12) aufweist oder aus dem unelastischen Abschnitt (12) besteht.

3. Stützbandage nach Anspruch 1, **dadurch gekennzeichnet, dass** der Stützgurt (10) einen flexiblen, unelastischen Abschnitt (12) und einen parallel zu dem unelastischen Abschnitt (12) angeordneten elastischen Abschnitt (13) aufweist und der unelastische Abschnitt (12) an dem elastischen Abschnitt (13) befestigt ist.

4. Stützbandage nach Anspruch 3, **dadurch gekennzeichnet, dass** der unelastische Abschnitt (12) permanent an dem elastischen Abschnitt (13) fixiert ist und eine Länge aufweist, die von der Länge des ungedehnten elastischen Abschnittes (13) verschieden ist.

5. Stützbandage nach Anspruch 3, **dadurch gekennzeichnet**, das der unelastische Abschnitt (12) reversibel an dem elastischen Abschnitt (13) festgelegt ist.

6. Stützbandage nach Anspruch 5, **dadurch gekennzeichnet, dass** der unelastische Abschnitt (12) über Formschlusselemente (23) an dem elastischen Abschnitt (13) festgelegt ist.

7. Stützbandage nach einem der Ansprüche 3 bis 6, **dadurch gekennzeichnet, dass** mehrere parallel zueinander angeordnete elastische und unelastische Abschnitte (12, 13) in Reihe zueinander angeordnet sind.

8. Stützbandage nach Anspruch 7, **dadurch gekennzeichnet, dass** zwischen zwei elastischen Abschnitten (13) ein unelastisches Gurtsegment (15) angeordnet ist.

9. Stützbandage nach einem der Ansprüche 3 bis 8, **dadurch gekennzeichnet, dass** der unelastische Abschnitt (12) kürzer oder länger als der ungedehnte elastische Abschnitt (13) ist.

10. Stützbandage nach einem der Ansprüche 3 bis 9, **dadurch gekennzeichnet, dass** mehrere elastische Abschnitte (13) parallel zueinander angeordnet sind.

11. Stützbandage nach einem der voranstehenden Ansprüche, **dadurch gekennzeichnet, dass** die Formschlusselemente (21) reversibel auf dem Grundkörper (2) festgelegt sind.

12. Stützbandage nach einem der voranstehenden Ansprüche, **dadurch gekennzeichnet, dass** in dem Grundkörper (2) eine Führung (8) für den Stützgurt (10) eingearbeitet ist.

13. Stützbandage nach einem der voranstehenden Ansprüche, **dadurch gekennzeichnet, dass** an dem Textilmaterial des Grundkörpers (2) oder dem Stützgurt (10) elastische Elemente befestigt oder Perforationen eingebracht sind, die eine bereichsweise Änderung der Elastizität der Stützbandage bewirken.

14. Stützbandage nach einem der voranstehenden Ansprüche, **dadurch gekennzeichnet, dass** der Stützgurt (10) zumindest teilweise auf der Innenseite des Grundkörpers (2) angeordnet ist.

## Claims

1. A support bandage comprising a base body (2) that is made of an elastic textile material and that has an inner side (3) facing toward the body of a bandage user and an outer side (4) facing away from the body of the bandage user, with an anti-slip coating (5) arranged on the inner side (3), **characterized in that** the support bandage (1) has at least one supporting strap (10), which is arranged reversibly on the outer side of the base body (2) by way of form-fitting elements (21) and that on the inner side facing toward the skin surface, the supporting strap (10) has an anti-slip coating (5).

2. The support bandage as claimed in claim 1, **characterized in that** the supporting strap (10) has at least one flexible, non-elastic section (12) or consists of the non-elastic section (12).

3. The support bandage as claimed in claim 1, **characterized in that** the supporting strap (10) has a flexible, non-elastic section (12) and an elastic section (13) arranged parallel to the non-elastic section (12) and the non-elastic section (12) is secured on the elastic section (13).

4. The support bandage as claimed in claim 3, **characterized in that** the non-elastic section (12) is fixed permanently on the elastic section (13) and has a length that is different than the length of the unstretched elastic section (13).

5. The support bandage as claimed in claim 3, **characterized in that** the non-elastic section (12) is fastened reversibly on the elastic section (13).

6. The support bandage as claimed in claim 5, **characterized in that** the non-elastic section (12) is fastened on the elastic section (13) by way of form-fitting elements (23).

7. The support bandage as claimed in one of claims 3 through 6, **characterized in that** several elastic and non-elastic sections (12, 13) arranged parallel to one another are arranged in series.

8. The support bandage as claimed in claim 7, **characterized in that** a non-elastic strap segment (15) is arranged between two elastic sections (13).

9. The support bandage as claimed in one of claims 3 through 8, **characterized in that** the non-elastic section (12) is shorter or longer than the unstretched elastic section (13).

10. The support bandage as claimed in one of claims 3 through 9, **characterized in that** several elastic sections (13) are arranged parallel to one another.

11. The support bandage as claimed in one of the preceding claims, **characterized in that** the form-fitting elements (21) are fastened reversibly on the base body (2).

12. The support bandage as claimed in one of the preceding claims, **characterized in that** a guide (8) for the supporting strap (10) is worked into the base body (2).

13. The support bandage as claimed in one of the preceding claims, **characterized in that** elastic elements are secured on, or perforations are introduced into, the textile material of the base body (2) or the supporting strap (10) and modify the elasticity of the support bandage in some areas.

14. The support bandage as claimed in one of the preceding claims, **characterized in that** the supporting strap (10) is arranged at least partially on the inner side of the base body (2).

## Revendications

1. Bandage de contention comprenant un corps de base (2) en matériau textile élastique, présentant un côté intérieur (3) tourné vers le corps de l'utilisateur du bandage et un côté extérieur (4) tourné à l'opposé du corps de l'utilisateur du bandage, avec un revêtement antidérapant (5) agencé sur le côté intérieur (3), **caractérisé en ce que** le bandage de contention (1) présente au moins une ceinture de contention (10) qui est agencée de façon réversible sur à l'extérieure du corps de base (2) par l'intermédiaire d'éléments à formes conjuguées (21), et **en ce que** sur le côté intérieur tourné vers la surface de la peau, la ceinture de contention (10) présente un revêtement antidérapant (5).

2. Bandage de contention selon la revendication 1, **caractérisé en ce que** la ceinture de contention (10) présente au moins une partie flexible inélastique (12) ou bien est constituée de la partie inélastique (12).

3. Bandage de contention selon la revendication 1, **caractérisé en ce que** la ceinture de contention (10) présente une partie flexible inélastique (12) et une partie élastique (13) disposée parallèlement à la partie inélastique (12), et la partie inélastique (12) est fixée à la partie élastique (13).

4. Bandage de contention selon la revendication 3, **caractérisé en ce que** la partie inélastique (12) est fixée de façon permanente à la partie élastique (13) et présente une longueur différente de celle de la partie élastique (13) non étirée.

5. Bandage de contention selon la revendication 3, **caractérisé en ce que** la partie inélastique (12) est fixée de façon réversible à la partie élastique (13).

6. Bandage de contention selon la revendication 5, **caractérisé en ce que** la partie inélastique (12) est fixée à la partie élastique (13) par l'intermédiaire d'éléments à formes conjuguées (23).

7. Bandage de contention selon l'une des revendications 3 à 6, **caractérisé en ce que** plusieurs parties élastiques et inélastiques (12,13) disposées parallèlement les unes aux autres sont agencées en série les unes par rapport aux autres.

8. Bandage de contention selon la revendication 7, **caractérisé en ce qu'**un segment inélastique de ceinture (15) est disposé entre deux parties élastiques (13).

9. Bandage de contention selon l'une des revendications 3 à 8, **caractérisé en ce que** la partie inélastique (12) est plus courte ou plus longue que la partie élastique (13) non étirée.

10. Bandage de contention selon l'une des revendications 3 à 9, **caractérisé en ce que** plusieurs parties élastiques (13) sont agencées parallèlement les unes aux autres.

11. Bandage de contention selon l'une des revendications précédentes, **caractérisé en ce que** les éléments à formes conjuguées (21) sont fixés de façon réversible sur le corps de base (2).

12. Bandage de contention selon l'une des revendications précédentes, **caractérisé en ce qu'**un guidage (8) est pratiqué dans le corps de base (2) pour la ceinture de contention (10).

13. Bandage de contention selon l'une des revendications précédentes, **caractérisé en ce qu'**au matériau textile du corps de base (2) sont fixés des éléments élastiques ou pratiquées des perforations, ayant pour effet une modification par zones de l'élasticité du bandage de contention.

14. Bandage de contention selon l'une des revendications précédentes, **caractérisé en ce que** la ceinture de contention (10) est au moins partiellement disposée sur le côté intérieur du corps de base (2).
